(19) Europäisches Patentamt
European Patent Office
Office européen des brevets

(11) **EP 3 264 137 A1**

(12) **EUROPEAN PATENT APPLICATION**

(43) Date of publication:
**03.01.2018 Bulletin 2018/01**

(51) Int Cl.:
***G01T 1/10*** (2006.01)     ***A61B 6/00*** (2006.01)
***A61N 5/10*** (2006.01)

(21) Application number: **16177569.7**

(22) Date of filing: **01.07.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Aarhus Universitet**
**8000 Aarhus C (DK)**
• **Region Midtjylland**
**8800 Viborg (DK)**

(72) Inventors:
• **Balling, Peter**
**8210 Aarhus V (DK)**

• **Høye, Ellen Marie**
**8000 Aarhus C (DK)**
• **Sadel, Michael**
**8000 Aarhus C (DK)**
• **Skyt, Peter Sandegaard**
**8270 Højbjerg (DK)**
• **Muren, Ludvig Paul**
**8240 Risskov (DK)**
• **Petersen, Jørgen Breede Baltzer**
**8200 Aarhus N (DK)**

(74) Representative: **Plougmann Vingtoft a/s**
**Rued Langgaards Vej 8**
**2300 Copenhagen S (DK)**

(54) **PHANTOM FOR THREE-DIMENSIONAL DOSIMETRY**

(57) There is presented a phantom (734) comprising a matrix and a plurality of particles embedded in said matrix, wherein said particles comprise optically stimulated luminescence material, wherein a size of the phantom along each and all of the three geometrical dimensions is at least 10 millimetre. In an embodiment, an attenuation coefficient of the phantom is equal to or less than 10 cm$^{-1}$ at a wavelength, where optical stimulation of the particles can be carried out at said wavelength and/or where stimulated luminescence takes place.

FIG. 2

Printed by Jouve, 75001 PARIS (FR)

EP 3 264 137 A1

## Description

FIELD OF THE INVENTION

The present invention relates to a dosimeter for measuring ionizing radiation, and more particularly to a phantom for three-dimensional dosimetry via optically stimulated luminescence and corresponding method for preparation and use.

BACKGROUND OF THE INVENTION

Radiotherapy is a treatment involving the use of radiation, in particular ionizing radiation. It may for example be used to treat cancer, where high-energy radiation used during radiotherapy permanently damages the DNA of cancer cells, causing them to die. Several three-dimensional dosimeters exist and rely on, e.g., polymerizing or radio-chromic gels, but have different shortcomings, e.g., not being re-usable, involving complicated readout, having physical properties (such as refractive index changes) leading to artefacts, being sensitive to oxidation, being unstable and/or having a dose-rate dependent response.

Hence, an improved three-dimensional dosimeter or phantom would be advantageous, and in particular it would be advantageous with a three-dimensional dosimeter or phantom which is re-usable, enables simplified readout, is less sensitive to oxidation, is stable, has dose-rate independent response and/or has physical properties enabling avoidance of artefacts.

SUMMARY OF THE INVENTION

It may be seen as an object of the present invention to provide a phantom and corresponding method for preparation and use that solves the above mentioned problems of the prior art with not being re-usable, involving complicated readout, having physical properties (such as refractive index changes) leading to artefacts, being sensitive to oxidation, being unstable and/or having a dose-rate dependent response

It is a further object of the present invention to provide an alternative to the prior art.

Thus, the above described object and several other objects are intended to be obtained in a first aspect of the invention by providing a phantom, such as a phantom suitable for three-dimensional dosimetry, comprising:

- a matrix,
- a plurality of particles embedded in said matrix, wherein said particles comprise optically stimulated luminescence material, such as said particles being radiation excitable (such as electrons in the particles may be brought into excited states via ionizing radiation) and capable of emitting optically stimulated luminescence (such as may be brought to luminesce via stimulation with electromagnetic radiation, such as incident electromagnetic radiation may trigger recombination of electrons in the excited states releasing energy as stimulated radiative luminescence),

wherein a size of the phantom along each and all of the three geometrical dimensions is at least 10 millimetre, such as at least 15 millimetre, such as at least 20 millimetre, such as at least 50 millimetre, such as at least 100 millimetre, such as at least 150 millimetre such as at least 200 millimetre.

The invention is particularly, but not exclusively, advantageous for providing a phantom (i.e., a model of a part of the human body), which can be subjected to radiation and which phantom can subsequently be subjected to examination revealing the dose distribution. This may for example be relevant for validating (before actually subjecting a patient to the radiation) that a dose distribution of radiation does in fact correspond to the area requiring treatment, while leaving healthy tissue as unaffected as possible.

An advantage of said phantom may be, that it enables benefitting from good dosimetric properties of typically relatively expensive optically stimulated luminescence (OSL) material in a relatively inexpensive way, since the phantom volume is furthermore occupied by potentially relatively inexpensive matrix material. It may be seen as an insight of the present inventors that the present invention may enable the well established, good dosimetric properties of OSL materials being combined with a potentially relatively inexpensive matrix of good optical properties (and optionally good mechanical properties with respect to the mechanical properties of the material of the particles), which matrix can be used to suspend particles of the preferred OSL material in a phantom of a desired 3D shape, which phantom has dimensions and optical properties, which makes it suitable for use as a three-dimensional dosimeter. It may be seen as another insight of the inventors that such combination of particles and matrix may surprisingly yield an increased photon count at a detector outside of the phantom with respect to a photon count resulting from a phantom consisting of particles (i.e., in the absence of the matrix) even though a phantom with particles only would comprise a higher mass of particles per volume. The matrix may not contribute to the dosimetric response; it may simply allow optical access to the embedded OSL-active particles.

Another advantage may be, that mechanical properties of said phantom with OSL particles may be at least partially decoupled from the mechanical properties of the material of the OSL particles, since mechanical properties can be controlled via the matrix. Another advantage may be that the phantom is re-usable since electron-hole pairs in the OSL material can be repeatedly excited by ionizing radiation and recombined by optical stimulation (i.e., OSL dosimeters can be reset by, e.g., high temperature and/or an optical bleaching procedure).

Another advantage may be that readout may be kept relatively simple, since the phantom may be scanned via optical stimulation and/or since the luminescence results in photons from the irradiated voxels, which may be directly imaged. Another possible advantage in terms of simplifying the readout is that irradiation does not change the refractive index. Another possible advantage may be relatively high stability and little or no sensitivity towards oxidation, which may potentially simplify storage and use. Another possible advantage may be dose-rate independent response, which may simplify data analysis. Another possible advantage is that readout may be carried out subsequent to irradiation, such as seconds, minutes, hours or days subsequent to irradiation, which may enable readout elsewhere than at the site of irradiation, which may in turn be beneficial for enabling irradiation, e.g., in a strong magnetic field where electronic devices are not applicable, and then subsequent readout without the strong magnetic field with electronic devices. Other possible advantages include good tissue equivalence and/or no dependence on external factors (such as humidity or temperature).

By 'phantom' is to be understood a mechanical model, such as a mechanical model of a human or animal body part, such as an anthropomorphic model, which phantom enables measuring a dose of irradiation which the phantom may be subjected to. More particularly, the phantom may change properties and/or emit radiation upon irradiation and optionally stimulation, which change in properties and/or emitted radiation may be indicative of the dose of irradiation. Said phantom may be nonbiological, such as not comprising living or dead tissue. Phantom may be employed interchangeably with "three-dimensional dosimeter".

By 'matrix' is to be understood a material in which something is enclosed or embedded. The matrix of the phantom may be understood to be solid, homogeneous, monolithic and/or polymeric.

By 'particles' may be understood solid structures which have relatively small volume and dimensions with respect to the volume and dimensions of the matrix material. The particles may comprise or consist of crystalline material. The particles may have dimensions, such as dimensions in each and all 3 geometrical directions, within one micrometer to hundreds of micrometers, such as within 50-200 micrometer, such as within 100-200 micrometer.

By 'embedded (in said matrix)' may be understood to enclose closely, such as to make the embedded structure(s) an integral part of the embedding structure, such as the matrix and the embedded particles forming an integral structure. By 'enclose closely' may be understood that there is little or substantially no, such as no, air (or other gas-filled) gap between the embedding (matrix) and embedded (particles) material, such as any gap is equal to or smaller than 1 times, such as 0.5 times, such as 0.25 times, such as 0.1 times, such as 0.01 times, the smallest wavelength of the optically stimulating wavelength and the wavelength of the photons emitted as optically stimulated luminescence.

'Optically stimulated luminescence' (OSL) is understood as is common in the art, more particularly as the emission of photons from an OSL material upon exposure to stimulating radiation. It may furthermore be understood that the excitation of the OSL material may take place via irradiation with ionizing radiation. Thus, OSL material is radiation excitable (such as electrons in the particles may be brought into trapped states via ionizing radiation) and capable of emitting optically stimulated luminescence (such as may be brought to luminesce via stimulation with electromagnetic radiation, such as incident electromagnetic radiation may trigger recombination of electrons from the trapped states releasing energy as stimulated radiative luminescence). Optically stimulated luminescence (OSL) employs dielectric crystalline materials with long-lived defect states (such as point defects in the crystal lattice, which are usually intentionally introduced by doping) in their band gaps, which can be populated by ionizing radiation. In OSL dosimetry, the population of these trapped states (corresponding to metastable, localized energy levels within the forbidden band, which are related to the defect states) may be read out by irradiating the sample with a moderately intense light source of appropriate wavelength to promote the trapped electrons to the conduction band and detecting the luminescence at shorter wavelength from, e.g., inter-band (electron-hole) recombination. The whole process is typically recorded by a photomultiplier tube (PMT) inside an OSL reader, which is equipped with light sources of wavelengths specified according to the OSL material, such as OSL particles, used. As a result, one can measure the time-dependent luminescent signal (and optionally the so-called OSL decay-curve), and from this evaluate the absorbed dose. Practical use of the OSL method may be facilitated by efficient light sources in the form of LEDs and/or lasers.

Materials which enable OSL may be interchangeably referred to as OSL materials, OSL active materials and/or optically stimulable luminescent materials. The particles of the present invention are understood to comprise, such as consist of, an OSL material.

By 'a size of the phantom along each and all of the three geometrical dimensions' may be understood that for at least one choice of Cartesian coordinate system, the largest length of the phantom along each of the three axes is at least a given size. In an embodiment, the phantom at least fills a volume corresponding to a solid cube with a side length corresponding to the given size. An advantage of having a size of at least 10 x 10 x 10 $mm^3$ may be that this matches a length scale, which is relevant for humans, and which may be resolved in three-dimensions, e.g., for voxels being 1 x 1 x 1 $mm^3$. An advantage of having a larger size may be that it enables matching length scales or larger structures or regions within a human.

In an embodiment, there is presented a phantom wherein an attenuation coefficient of the phantom is equal to or less than 10 cm$^{-1}$, such as equal to or less than 6.9 cm$^{-1}$, such as equal to or less than 5.0 cm$^{-1}$, such as equal to or less than 2.0 cm$^{-1}$, such as equal to or less than 1.4 cm$^{-1}$, such as equal to or less than 1.0 cm$^{-1}$, such as equal to or less than 0.9 cm$^{-1}$, at a wavelength, where optical stimulation of the particles can be carried out and/or at a wavelength where stimulated luminescence takes place. An advantage of this may be that it enables getting photons in to and/or out of an interior voxel, such as a voxel lying at least 1 cm from an outer interface of the phantom, while still carrying information regarding their initial direction.

By 'attenuation coefficient' may be understood Napierian attenuation coefficient u, such as wherein transmission Tthrough a material is given as:

$$T = \exp(-\mathrm{int}(u(z)\mathrm{d}z),$$

where *exp* denotes the exponential function, *int* denotes an integral (through the length of the material), z denotes a corresponding axis through the material and the corresponding coordinate).

The attenuation coefficient may be obtained as is common in the art, such as via measurement in a standard spectrophotometer, which measures the absorption though, e.g., a 1 cm cuvette. The measured absorbance, denoted by A, is in a standard apparatus determined as $A=\log(I_0/I)$, where log is the base-10 logarithm, $I_0$ is the intensity before the cuvette and $I$ the intensity after the cuvette. The measured absorbance is thus related to the Napierian attenuation coefficient as $A=\log(e) \mathrm{int}(u(z)\mathrm{d}z$ with e=2.71828 denoting the base number for the natural logarithm.

In a further embodiment, there is presented a phantom wherein said wavelength is within 200-1200 nm, such as within 200-1100 nm, such as within 400-1100 nm, such as within 400-1000 nm, such as within 400-800 nm. An advantage of this may be that the wavelength matches a wavelength wherein at least some relevant matrix materials (such as polymers) have good optical properties. An advantage of the 400-800 nm region may be that it matches the optically visible region, which has traditionally been employed in medical settings.

In an embodiment, there is presented a phantom wherein the phantom is arranged so as to enable that upon 1 Gy irradiation of the phantom with ionizing radiation, such as wherein said ionizing radiation is beta radiation, then at least 4000 photons can be emitted, such as emitted within 1 second upon stimulation with a Risoe TL/OSL DA-20 OSL reader (such as using an intensity of ~80 mW/cm$^2$ at the sample), from a voxel of 1 x 1 x 1 mm$^3$ via stimulated luminescence and traverse at least 1 cm of the phantom before reaching a detector. An advantage of this may be that a sufficient number of photons may then be received at a detector for the purpose of having sufficiently good statistics, such as achieving a statistical spread of approximately 2 %. In an embodiment, there is presented a phantom wherein the phantom is arranged so as to enable that upon 1 Gy irradiation of the phantom with ionizing radiation, such as wherein said ionizing radiation is beta radiation, then at least 1000 photons, such as at least 2000 photons, such as at least 3000 photons can be emitted, such as emitted within 1 second upon stimulation with a Risoe TL/OSL DA-20 OSL reader (such as using an intensity of ~80 mW/cm$^2$ at the sample), from a voxel of 1 x 1 x 1 mm$^3$ via stimulated luminescence and traverse at least 1 cm of the phantom before reaching a detector. An advantage of this may be that a sufficient number of photons may then be received at a detector for the purpose of having sufficiently good statistics, such as achieving a statistical spread of approximately 3 % (which is typically required for clinical purposes) corresponding to 1000 particles. By 'the phantom is arranged' may be understood an interplay between optical properties of matrix material and optical properties of the particles (including their OSL properties) and the optical properties of each of the matrix and the particles, such as having chosen matrix and particles which enable yielding a sufficient OSL response and which simultaneously enables optical transmission through the matrix as such and the composition comprising matrix and particles. It is noted that 1 cm may be a typical length scale of human anatomical structures and 1 mm$^3$ may be typical size of a voxel within the field of 3D dosimetry.

In an embodiment, there is presented a phantom wherein a density of said particles in said matrix is within 0.00001 to 0.0010 g/mm$^3$, such as within 0.00003 to 0.0001 g/mm$^3$, such as such as within 0.00003 to 0.00006 g/mm$^3$ or such as such as within 0.00004 to 0.00009 g/mm$^3$. An advantage of this may be that it enables providing a good OSL signal (such as sufficient number of photons emitted from the particles) while at the same time providing good optical properties (i.e., low attenuation coefficient), which in turn enables that a good number of optically stimulated luminescence photons can reach a detector, even if originating from voxels within the phantom.

In an embodiment, there is presented a phantom wherein the material of the phantom has a Young's Modulus of 2 megapascal or less, such as within ]0;2] MPa, such as within [2; 1000] kPa, such as within [2; 600] kPa, such as within [2; 35] kPa and/or within [30; 600] kPa. An advantage of this may be that it enables mimicking the mechanical properties of body parts, such as human body parts, which have Young's modules ranging between 0.001 and several megapascal. Another possible advantage may be that it enables that the phantom may be deformed during irradiation, such as giving possibilities to simulate organ deformation during irradiation in realistic geometries.

In an embodiment, there is presented a phantom wherein said matrix comprises silicone, such as a silicone elastomer, such as Sylgard® 184 (Dow Corning), and/or wherein said particles comprise lithium fluoride (LiF) doped with magnesium, copper and phosphorus (LiF:Mg,Cu,P), such as wherein the lithium fluoride (LiF) doped with magnesium, copper and phosphorus is enriched (with respect to natural Li) with lithium-6 isotope, with lithium-7 isotope or an other neutron converter material. An advantage of enriching or having another neutron converter material may be that it increases the cross-section with respect to neutrons, hence enables detection of neutron radiation. Neutron converters are described by E.G. Yukiharaa et al. in the reference Radiation Measurements 43 (2008) 309-314 which is hereby incorporated by reference in entirety. LiF:Mg,Cu,P may be referred to as "MCP". The matrix may be given by a polymeric material such as, polydimethylsiloxane silicones (PDMS), such as the Silicone Elastomer Kit SYLGARD® 184 consisting of preweighed monomer (base) and curing agent. Silicone may be understood as is common in the art, more particularly as an inorganic silicon-oxygen backbone chain (...-Si-O-Si-O-Si-O-...) with organic side groups attached to the silicon atoms. Silicones have in general the chemical formula $[R_2SiO]_n$, where $R$ is an organic group, such as methyl, ethyl, or phenyl.

In an embodiment, there is presented a phantom further comprising a radiochromic dye, such as leucomalachite green, embedded within said matrix. An advantage of this may be that the radiochromic response can then be used as an additional dosimetric measurement or simply as a visual aid for operators that the dosimeter was irradiated.

In an embodiment, there is presented a phantom wherein a ratio between the refractive indices of the matrix material and the particles at a wavelength, where optical stimulation of the particles can be carried out and/or at a wavelength where stimulated luminescence takes place, is equal to or less than 1.05, such as equal to or less than 1.04, such as equal to or less than 1.03, such as equal to or less than 1.02, such as equal to or less than 1.015, such as equal to or less than 1.01. An advantage of this may be that the relatively low ratio ensures a relatively low amount of refraction and scattering at the boundaries between the matrix material and the particles. For the purposes of the ratio calculation, it is to be understood that it is always equal to or larger than unity. It may be seen as an insight of the inventors, that the optical properties of the phantom, such as the attenuation coefficient, may depend not only on the (isolated) optical properties of the matrix as such and/or the optical properties of the matrix as such, but also on the relation between the optical properties of the matrix and the particles, such as the ratio of their refractive indices, since increasing this ratio may decrease the attenuation coefficient of the phantom, regardless of the respective attenuation coefficients of the matrix and the particles.

According to a second aspect of the invention, there is provided a method for preparing a phantom according to the first aspect, the method comprising, such as comprising the following steps in the sequence in which they are listed:

- Providing the particles,
- Providing the matrix material in liquid form,
- Mixing the particles and the matrix material while the matrix material is in liquid form,
- Allowing the matrix material to solidify.

This aspect of the invention is particularly, but not exclusively, advantageous in that it enables a relatively simple way of preparing the phantom. A possible advantage is that the method may relatively easily lend itself to moulding of the phantom, such as moulding into anthropomorphic shapes, while simultaneously benefitting from the optical properties of the particles, which may not be as easily mouldable. An advantage of mixing the particles and the matrix material while the matrix material is in liquid form may be that it dispenses with a need to implement OSL material into a solid matrix.

In an embodiment, there is presented a method for preparing a phantom and for estimating three-dimensional dose distribution of an ionizing radiation, wherein said estimating comprises

- Exposing the phantom to the ionizing radiation,
- Irradiating the phantom with electromagnetic radiation or heating the phantom,
- Mapping three-dimensional spatial distribution of stimulated luminescence,
- Estimating three-dimensional dose distribution based on said mapping.

According to a third aspect of the invention, there is provided use of a phantom according to the first aspect and/or as prepared according to the second aspect for any one of:

a. three-dimensional radiation dosimetry, such as for estimating three-dimensional dose distribution of an ionizing radiation, such as for estimating three-dimensional dose distribution of energetic subatomic particles, ions or atoms moving at high speeds (such as greater than 1% of the speed of light) and/or electromagnetic waves (such as within the high-energy end of the electromagnetic spectrum),
b. three-dimensional measurement of linear energy transfer (LET).

'Three-dimensional (3D) radiation dosimetry' is understood as is common in the art, more particularly as the process of experimental (as opposed to theoretical calculations or numerical simulations) determination of the spatial distribution of the absorbed dose of ionising radiation, more particularly exposure of a three-dimensional dosimeter, such as phantom, to the ionizing radiation and subsequently estimating three-dimensional (3D) dose distribution by measuring changes in the dosimeter, which correlates with the amount of ionizing radiation. 'Linear energy transfer' (LET) is understood as is common in the art, such as is described by Granville et al. in the reference Physics in Medicine and Biology, Vol. 61, p. 1765-1779 (2016) which is hereby incorporated by reference in entirety.

'Ionizing radiation' is understood as is common in the art, more particularly to include energetic subatomic particles, ions or atoms moving at high speeds (such as greater than 1% of the speed of light) and/or electromagnetic waves (such as within the high-energy end of the electromagnetic spectrum).

In order to read out the 3D dose distribution, different optical techniques can be employed. First, in some cases (at high doses), it may be possible that the population of the defect states are directly observable in an absorption measurement. In this case, the dose distribution can be read out using standard optical CT scanners. Alternatively, the read out can be based on an appropriately designed combination of an illuminating light source and an optical-detection setup for the luminescent light, which together determines the 3D distribution. In one embodiment, the illuminating light source is a laser, which illuminates a specific part of the dosimeter and the detection is based on an appropriate optical filter in combination with a photo multiplier tube (PMT), an image-intensified camera (ICCD) or an electron-multiplication camera (EMCCD).

In an embodiment, there is presented use comprising optical stimulation

> a. at a plurality of wavelengths, and/or
> b. according to a plurality of different stimulation modes, such as said stimulation modes being any one of continuous, pulsed, linear.

Stimulation modes being 'continuous, pulsed, linear' is understood as is common in the art and more particularly as continuous wave OSL, or CW-OSL, pulsed-OSL (POSL), linear modulation OSL, or LM-OSL as described by M.S. Akselrod et al. in Radiation Measurements 41 (2007) S78 - S99 which is hereby incorporated by reference in entirety.

In an embodiment, there is presented use for three-dimensional radiation dosimetry, such as estimating three-dimensional dose distribution of an ionizing radiation, wherein said use comprises,

- Exposing the phantom to the ionizing radiation, such as so as to excite electrons in the phantom,
- Irradiating the phantom with electromagnetic radiation, such as so as to optically stimulate luminescence,
- Mapping three-dimensional spatial distribution of stimulated luminescence, such as determining an amount of stimulated luminescence from individual voxels within a plurality of voxels within the phantom wherein said stimulated luminescence is at least partially received from voxels from which photons have to pass other voxels to reach a detector, such as mapping three-dimensional spatial distribution of the luminescing particles,
- Estimating three-dimensional dose distribution based on said mapping.

In embodiments, at least some (internal) voxels, for which the dose has to be estimated, both the stimulating radiation and the stimulated luminescence travels through other voxels (between internal voxels and, respectively, a source of stimulating radiation and a detector) for which other voxels the dose also has to be estimated. This may be advantageous for dispensing with a need for physically disassembling, such as physically slicing, the phantom. In alternative embodiments, irradiating the phantom with electromagnetic radiation, such as so as to optically stimulate luminescence, may be partially or fully replaced with heating the phantom, such as so as to thermally stimulate luminescence.

By 'mapping' may be understood obtaining information regarding the spatial origin within the phantom of the stimulated luminescence. Such mapping may thus be employed to determine spatially, in three dimensions, where (such as from which voxels), the stimulated luminescence originates. The information regarding the spatial origin may be obtained, e.g., by scanning the phantom with stimulating electromagnetic radiation and/or using imaging optics which not only detects a photon, but also obtain information regarding its origin, such as from which voxel if was emitted.

By 'estimating three-dimensional dose distribution based on said mapping' may be understood that the information gained from the mapping may be used to obtain information on dose distribution, since the amount of stimulated luminescence may correlated with the dose of ionizing radiation.

In an embodiment, there is presented use wherein said irradiating comprises:

- Scanning the phantom with the electromagnetic radiation, such as said scanning involves scanning in 3 dimensions, such as sequential scanning of individual points or voxels,

and wherein said mapping comprises for a plurality of scanning positions:

- Registering corresponding values of coordinates of said scanning and intensity of luminescence.

In an embodiment, there is presented use wherein the scanning comprises:

- Scanning the phantom along a scanning direction with a beam of light forming a sheet of light, wherein a surface normal of said sheet of light is non-parallel with the scanning direction,

and wherein said registering comprises for the plurality of scanning positions:

- Registering corresponding values of coordinates of said scanning and intensity of luminescence spatially resolved in a plane comprising the light sheet.

For example, the illuminating laser can illuminate a specific plane of a thickness below 1 mm and a camera can image the luminescence light from this plane (from a position outside of the plane). In this way, the two-dimensional dose distribution of said plane can be measured, and by shifting said plane relative to the dosimeter, the 3D dose distribution can be obtained by building up stacks of 2D distributions. An advantage of this (as well as for scanning individual voxels with the stimulating radiation) may be that it dispenses with a need for employing any inversion algorithms.

The first, second and third aspect of the present invention may each be combined with any of the other aspects. These and other aspects of the invention will be apparent from and elucidated with reference to the embodiments described hereinafter.

BRIEF DESCRIPTION OF THE FIGURES

The phantom, method and use according to the invention will now be described in more detail with regard to the accompanying figures. The figures show specific ways of implementing the present invention and is not to be construed as being limiting to other possible embodiments falling within the scope of the attached claim set.

Figure 1 shows OSL decay-curves.

Figure 2 shows a phantom which has been prepared in a 1 cm x 1 cm x 5 cm cuvette.

Figure 3 shows refractive indices of matrix and particles.

Figure 4 shows the attenuation of matrix and matrix with particles.

Figure 5 shows attenuation of a sample with silicone and particles.

Figure 6 shows a microscope image of a cross-sectional plane within the cuvette as depicted in figure 2.

Figure 7 shows a schematic of a setup for carrying out a method.

DETAILED DESCRIPTION OF EMBODIMENTS

EXAMPLE 1

Dosimeters of a shape appropriate for a commercial OSL reader have been prepared, more particularly dosimeters with a diameter of 7-8 mm and thickness below 1 mm cast directly on a small metal disk. The dosimeter samples were made in standard OSL aluminium trays carrying a volume corresponding to a circular disc of 8 mm diameter times ~1 mm thickness.

The particles comprising OSL material are given by particles of lithium fluoride (LiF) doped with magnesium, copper and phosphorus (LiF:Mg,Cu,P), also known as "MCP". The matrix is given by a silicone elastomer (more particularly Polydimethylsiloxane elastomer) and curing agent, Sylgard® 184 Silicone Elastomer Kit (Dow Corning). The curing agent and the silicone elastomer were used in a 1:10 weight ratio. Silicone was first mixed with the curing agent and next with the LiF:Mg,Cu,P luminescence powder. Air bubbles were removed from the dosimeter by use of a vacuum desiccator. The samples were mixed before being poured into the disks. The disks were standard used in commercial OSL readers metal trays with a diameter between 7-8 mm and thickness 1 mm. The dosimeters were placed for 30 minutes in an oven kept at 60 degrees centigrade to accelerate the curing process.

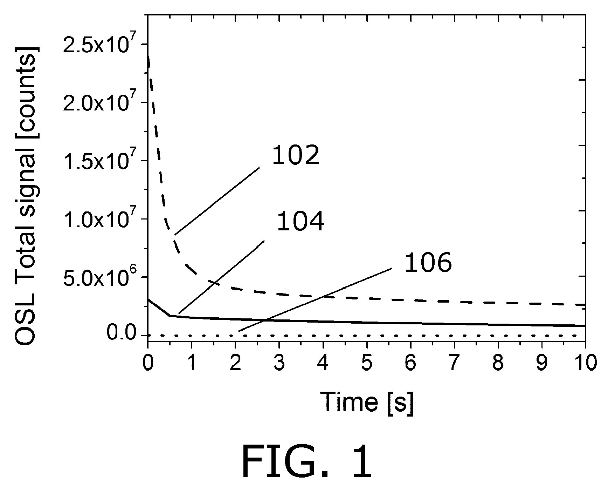

Figure 1 shows OSL decay-curves showing the number of counts in 0.4 s time bins from different samples with MCP and/or silicone obtained by irradiating with a Beta source with a dose of 1 Gy. The OSL was stimulated with blue light at a room temperature using a commercial OSL reader with UV filter and a PMT. In Fig. 1 the OSL decay curves of different samples, obtained after irradiations with Beta radiation are compared. The total beta dose was at the level of 1 Gy in all cases. The OSL read outs were performed using a Risoe TL/OSL DA-20. The samples were stimulated with blue light emitting diodes (LEDs), with emission centered at 470 nm and an intensity of ~80 mW/cm$^2$ at the sample. A Hoya UV-340 filter of 7.5 mm thickness and Ø = 45 mm were used.

The stimulation wavelength range for MCP powder is 450-550 nm and the luminescence wavelengths are 250-425 nm (as taken from the data for the Risoe reader employing a UV-340 optical filter with main transmission between 250-400 nm, and blue light emitting diodes 470 nm for stimulation, as mentioned above).

For the analyses using the OSL signal, the period of stimulation was 100 s. The dosimeter samples were made in standard OSL aluminium trays carrying a volume corresponding to a circular disc of 8 mm diameter times ~1 mm thickness. The measurement shows that the OSL signal from MCP embedded in silicone (shown in the upper, dashed curve 102) was significantly higher than that of MCP powder (shown in the middle, full drawn curve 104). The lower, dotted curve 106 shows the signal for an empty probe. We note that the two samples used approximately the same amount of dosimeter material (0.045 g for pure MCP powder; 0.068 g for MCP in silicone). The higher response may be due to an improved emission of the OSL light from the embedded (optically contacted) particles compared to the translucent powder.

It is also noted that the silicone matrix in itself does not add to the OSL signal.

As it is can be seen in Fig. 1, on a commercial OSL reader using appropriate filters and a PMT, a sample according to EXAMPLE 1 with combined silicone and MCP powder (as depicted in the upper, dashed curve 102) gives ~4·10$^7$ counts in the first second from a standard-size test sample (MCP powder + silicone) after a dose of 1 Gy (this can be seen in the FIG. 1). This signal originates from an area of pi·(4 mm)$^2$, i.e. 50 mm$^2$. From the mass of the sample, 0.068 g, and the density of silicone, ~1.03 g/cm3, the thickness can be estimated as ~1.3 mm - comparable to an appropriate thickness of a light sheet in 3D read out. The luminescence light may possibly be reflected from the metal disk behind the dosimeter material (giving approximately a factor of two higher collection efficiency). We thus estimate that the expected signal from a 1 mm thick slab of a 3D dosimeter will be down by a factor of two. This means that - for a dose of 1 Gy - we should expect ~4-10$^7$/50/2 = 400,000 counts from each 1 mm$^3$ voxels. The solid angle in the commercial OSL reader is quite high, covering maybe light emitted within ±45° from the sample (i.e. an N.A. of ~0.7 equivalent to an f number (f#) of f/0.7). It may be difficult to match this collection efficiency with an imaging setup, although fairly-large-lensed objectives are available, e.g. telecentric lenses with f/2.8. We may thus expect to lose some light (e.g. a factor of 4) compared to the PMT setup. On the other hand, the detection efficiency in a state-of-the-art EMCCD (single-photon camera) is similar to (or even higher than) that of a PMT, so we should still have a large dynamic range covering the clinically relevant doses. Taking into account that the obtained OSL signal from the silicone and MCP powder sample of EXAMPLE 1 corresponds to 1 Gy and remembering that a typical dose fraction in for example proton radiotherapy is at the level of several to approximately ten Gys, the dosimeter material is applicable for real dosimetric applications. Note also that the response of MCP to beta radiation is known to be lower than to gamma radiation. This issue is commonly known as the luminescence efficiency. For weakly ionizing radiation (e.g. photons), the luminescence efficiency must be assumed as equal unity. For strongly ionizing radiation (particles), the efficiency usually decreases. Consequently, for the same doses of beta and gamma radiation, the response (signal) from the detector will be lower for beta particles than for photons. This effect is especially observable for heavy charged particles like protons. Taking this into account, we should expect even higher signal for gamma radiation comparing to the beta data on the figure 1. On the other hand, for protons, the signal levels will be similar to those for beta radiation. Signal levels above were calculated over one second although more OSL light could be acquired if the full OSL decay curve is measured. Note that the signal per unit time depends on the intensity of the exciting light source.

It is noted that in the embodiment of the invention using a light sheet, we can estimate the laser power needed to illuminate such a sheet: a 1 mm thick times 200 mm high sheet irradiated to an intensity similar to the commercial OSL reader (80 mW/cm$^2$), requires a laser power of only approximately 160 mW, which is readily available. In other words, a dose distribution from a 1-mm-thick slice of the dosimeter can be acquired in about 1 second. If the dosimeter has a thickness of 200 mm, the full 3D dose distribution can thus be scanned in 200 seconds, or 3.5 minutes. After scanning no computational inversion is required in order to obtain dose distribution.

EXAMPLE 2

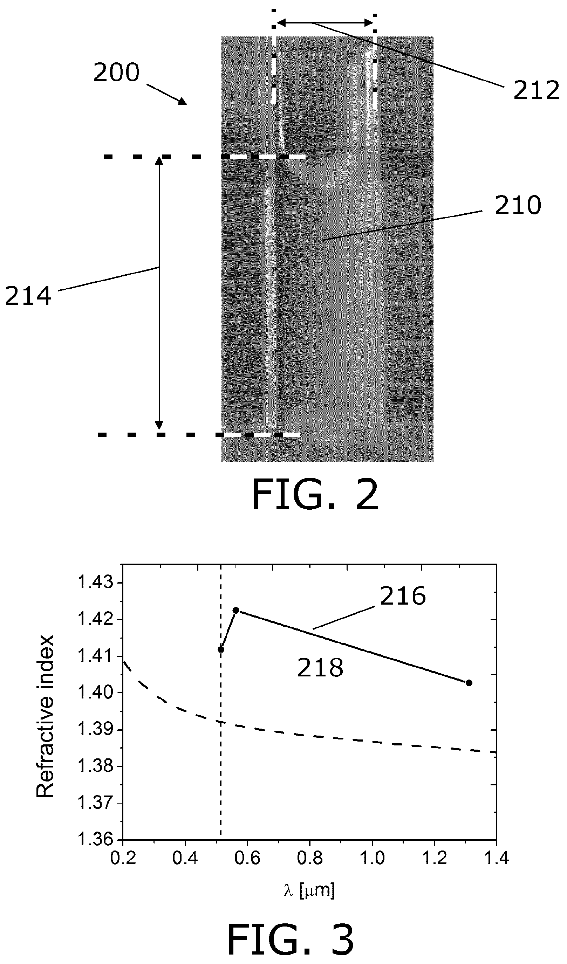

Figure 2 shows a phantom 210 which has been prepared in a 1 cm x 1 cm x 5 cm cuvette 200. As in EXAMPLE 1, the material choices are MCP microcrystals (as the OSL particles) embedded in a matrix given as silicone elastomer and curing agent, Sylgard® 184 Silicone Elastomer Kit (Dow Corning). The width 212 of the phantom is thus 1 cm and the height 214 is approximately 3.5 cm. The curing agent and the silicone elastomer were used in a 1:10 weight ratio. Silicone was first mixed with the curing agent and next with the LiF:Mg,Cu,P luminescence powder. Air bubbles were removed from the dosimeter by use of a vacuum desiccator. The samples were mixed before being poured into the appropriate cuvettes. The cuvettes were standard PMMA cuvettes 1 cm x 1xm x 5 cm. They were subsequently placed into the oven to accelerate curing process as in Example 1.

For the preparation of the phantom 0.15-0.3 g of MCP powder was used for 5 1m$^3$ corresponding to 0.00003-0.00006 g/mm$^3$. It is noted that this is about 2 - 4 % of that used in EXAMPLE 1. A factor of 25 - 50 times lower signal levels from the 3D dosimeter with the EXAMPLE 2 particle concentration is thus expected with respect to EXAMPLE 1. This is still more than 10,000 counts (i.e. statistical spread of 1%). Note that for a normal distribution with sample size (number of photons) N, the statistical spread for the measurements is square root (sqrt) of N, so the relative spread is 1/sqrt(N) = 1 % when N=10,000. Required clinical accuracy is typically a statistical spread of approximately 3 %.

It can be readily seen in figure 2, that the phantom is transparent to the naked eye. This transparency may at least partially be caused by index matching of the refractive indices of the matrix and the particles.

Figure 3 shows the refractive index of the matrix 216 (in the form of the refractive index of the silicone as employed in EXAMPLES 1-2) and LiF 218 (i.e., lithium fluoride, which forms the base material of MCP, which is employed for the particles in EXAMPLES 1-2).

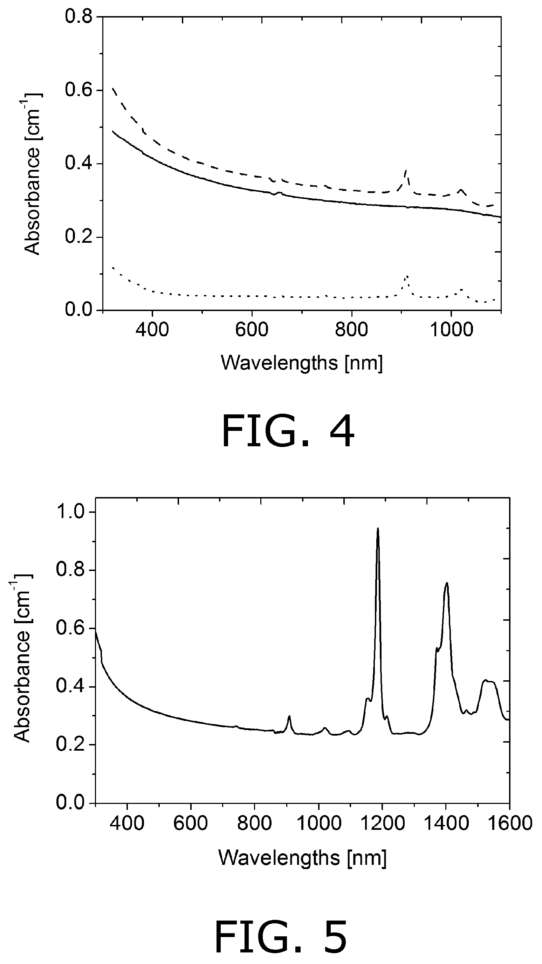

Figure 4 shows the absorbance of (in the lower curve) the silicone employed in EXAMPLES 1-2 (without particles), (in the middle curve) the silicone with the particles as provided in EXAMPLE 2 where the silicone attenuation has been subtracted, and (in the top curve) the silicone with the particles as provided in EXAMPLE 2.

Figure 5 shows absorbance of a sample with silicone and particles.

The size of the MCP particles in EXAMPLES 1-2 is from 100 - 200 micrometer. Therefore it may be presumed to have a maximum of around 100 single grains in a 1mm$^3$ voxel. For the current 3D versions (EXAMPLE 2), the mass concentration of MCP ~0.00003 g/mm$^3$ indicates a few particles per 1 mm$^3$ voxel.

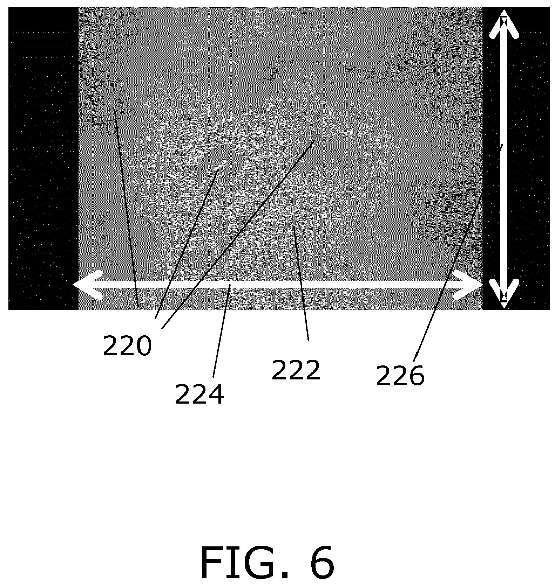

Figure 6 shows a microscope image of a cross-sectional plane within the cuvette as depicted in figure 2, where particles 220 and matrix 222 can be seen. The image width 224 is 1.1 mm and the image height 226 is 0.9 mm. From such images taken at different cross-sections, it can be derived, that the number of particles is 10-20 per mm$^3$ in the middle of the cuvette.

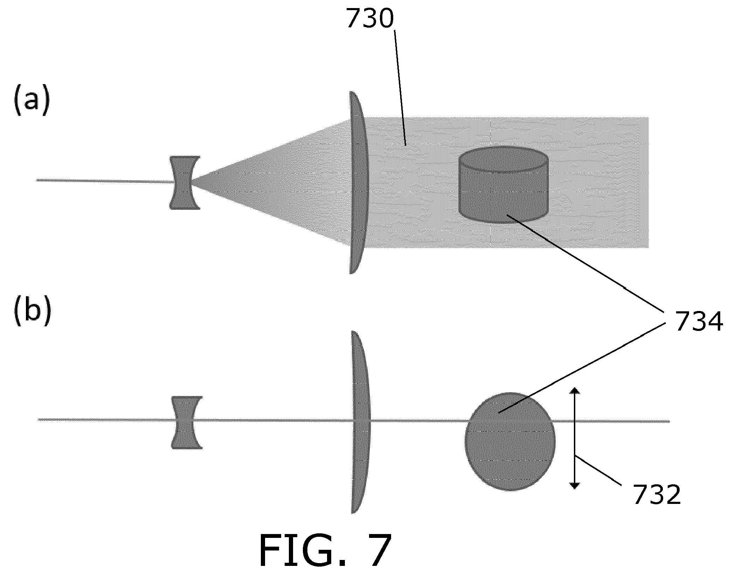

Figure 7 shows a schematic of a setup for carrying out a method, more particularly the method wherein scanning comprises:

- Scanning the phantom along a scanning direction with a beam of light forming a sheet of light, wherein a surface normal of said sheet of light is non-parallel with the scanning direction,

and wherein said registering comprises for the plurality of scanning positions:

- Registering corresponding values of coordinates of said scanning and intensity of luminescence spatially resolved in a plane comprising the light sheet.

The figure shows a side view in sub-figure (a) and a top view in sub-figure (b). The figures depict a laser beam 730 forming a sheet of light, which beam scanning (as indicated by arrow 732) a phantom 734.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

**Claims**

1. A phantom (734), such as a phantom for three-dimensional dosimetry, comprising:

- a matrix,
- a plurality of particles embedded in said matrix, wherein said particles comprise optically stimulated luminescence material,

wherein a size of the phantom along each and all of the three geometrical dimensions is at least 10 millimetre.

2. A phantom (734) according to any one of the preceding claims, wherein an attenuation coefficient of the phantom is equal to or less than 10 cm$^{-1}$ at a wavelength, where optical stimulation of the particles can be carried out at said wavelength and/or at a wavelength where stimulated luminescence takes place.

3. A phantom (734) according to any one of the preceding claims, wherein said wavelength is within 200-1200 nm.

4. A phantom (734) according to any one of the preceding claims, wherein the phantom is arranged so as to enable that that upon 1 Gy irradiation of the phantom with ionizing radiation then at least 4000 photons can be emittedfrom a voxel of 1 x 1 x 1 mm$^3$ via stimulated luminescence and traverse at least 1 cm of the phantom before reaching a detector.

5. A phantom (734) according to any one of the preceding claims, wherein a density of said particles in said matrix is within 0.00001 to 0.0010 g/mm$^3$.

6. A phantom (734) according to any one of the preceding claims, wherein the material of the phantom has a Young's Modulus of 2 mega pascal or less.

7. A phantom (734) according to any one of the preceding claims, wherein said matrix comprises silicone.

8. A phantom (734) according to any of the preceding claims, wherein said particles comprise lithium fluoride doped with magnesium, copper and phosphorus.

9. A phantom (734) according to any one of the preceding claims, wherein a ratio between the refractive indices of the matrix material and the particles at a wavelength, where optical stimulation of the particles can be carried out and/or at a wavelength where stimulated luminescence takes place, is equal to or less than 1.05.

10. Method for preparing a phantom (734) according to any one of the preceding claims, the method comprising:

- Providing the particles,
- Providing the matrix material in liquid form,
- Mixing the particles and the matrix material while the matrix material is in liquid form,
- Allowing the matrix material to solidify.

11. Method for preparing a phantom (734) according to claim 10 and furthermore for estimating three-dimensional dose distribution of an ionizing radiation, wherein said estimating comprises:

- Exposing the phantom to the ionizing radiation,
- Irradiating the phantom with electromagnetic radiation or heating the phantom,
- Mapping three-dimensional spatial distribution of stimulated luminescence,
- Estimating three-dimensional dose distribution based on said mapping.

12. Use of a phantom (734) according to any one claims 1-9 for any one of:

a. three-dimensional radiation dosimetry,
b. three-dimensional measurement of linear energy transfer.

13. Use according to claim 12 for three-dimensional radiation dosimetry, such as estimating three-dimensional dose distribution of an ionizing radiation, wherein said use comprises,

- Exposing the phantom (734) to the ionizing radiation,
- Irradiating the phantom with electromagnetic radiation,
- Mapping three-dimensional spatial distribution of stimulated luminescence,
- Estimating three-dimensional dose distribution based on said mapping.

14. Use according to claim 13, wherein said irradiating comprises:

- Scanning the phantom (734) with the electromagnetic radiation, and wherein said mapping comprises for a plurality of scanning positions:
- Registering corresponding values of coordinates of said scanning and intensity of luminescence.

15. Use according to claim 14, wherein the scanning comprises:

- Scanning the phantom (734) along a scanning direction with a beam of light forming a sheet of light, wherein a surface normal of said sheet of light is non-parallel with the scanning direction,

and wherein said registering comprises for the plurality of scanning positions:

- Registering corresponding values of coordinates of said scanning and intensity of luminescence spatially resolved in a plane comprising the light sheet.

FIG. 1

200

212

210

214

## FIG. 2

216

218

Refractive index

λ [μm]

## FIG. 3

## FIG. 4

## FIG. 5

FIG. 6

(a)

730

(b)

734

732

FIG. 7

EP 3 264 137 A1

Europäisches
Patentamt

European
Patent Office

Office européen
des brevets

## EUROPEAN SEARCH REPORT

Application Number

EP 16 17 7569

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RUIQI LONG ET AL: "Design of an Optical System for Interrogation of Implanted Luminescent Sensors and Verification with Silicone Skin Phantoms", IEEE TRANSACTIONS ON BIOMEDICAL ENGINEERING, IEEE SERVICE CENTER, PISCATAWAY, NJ, USA, vol. 59, no. 9, 1 September 2012 (2012-09-01), pages 2459-2465, XP011490182, ISSN: 0018-9294, DOI: 10.1109/TBME.2012.2203306 | 10 | INV. G01T1/10 A61B6/00 A61N5/10 |
| Y A | * page 2460 - page 2461, left-hand column; figures * | 1-3,5-7 9,11, 13-15 | |
| X | RUAN CHUN ET AL: "Determination of multislice computed tomography dose index (CTDI) using optically stimulated luminescence technology", MEDICAL PHYSICS, AIP, MELVILLE, NY, US, vol. 37, no. 7, 15 June 2010 (2010-06-15), pages 3560-3568, XP012144828, ISSN: 0094-2405, DOI: 10.1118/1.3455285 * page 3561, paragraph II.A - page 3562, paragraph II.B; figures * | 1,4,8,12 | |
| | | | TECHNICAL FIELDS SEARCHED (IPC) |
| | | | G01T A61N A61B |
| Y | THOMPSON, HIRST, ANDREWS: "Is there a difference between laser speckle and laser Doppler in depthsensitivity?", SPIE, PO BOX 10 BELLINGHAM WA 98227-0010, USA, vol. 7897, 2011, XP040554055, * page 2, paragraph 2. * | 1-8,10, 12 | |

-/--

The present search report has been drawn up for all claims

| Place of search | Date of completion of the search | Examiner |
|---|---|---|
| The Hague | 21 December 2016 | Eberle, Katja |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

**EUROPEAN SEARCH REPORT**

Application Number

EP 16 17 7569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ROLF B. SAAGER ET AL: "Multilayer silicone phantoms for the evaluation of quantitative optical techniques in skin imaging", OPTICAL SENSING II, vol. 7567, 11 February 2010 (2010-02-11), page 756706, XP055329827, 1000 20th St. Bellingham WA 98225-6705 USA ISSN: 0277-786X, DOI: 10.1117/12.842249 ISBN: 978-1-62841-971-9 * abstract; figures; table 1 * * page 2, paragraph methods and materials * * page 3, paragraph; figure 2 * * page 4, paragraph 1 * | 1-8,10, 12 | |
| A | VASILIEV, GAMALIY, ZAYTSEV: "CT vs. volumescope: image quality and dose comparison", SPIE, PO BOX 10 BELLINGHAM WA 98227-0010 USA, vol. 5943, 2005, XP040215220, * abstract; figures * * page 6, paragraph 4. * | 11,13-15 | |
| Y | BENGT E BJARNGARD ET AL: "LITHIUM FLUORIDE-TEFLON THERMOLUMINESCENCE DOSIMETERS", LUMINESCENCE DOSIMETRY. PROCEEDINGS OF INTERNATIONAL CONFERENCE ON LUMINESCENCE DOSIMETRY, STANFORD UNIVERSITY, STANFORD, CALIFORNIA, JUNE 21-23, 1965, WASHINGTON, U.S.<PUBATTR/>,, 21 June 1965 (1965-06-21), pages 308-316, XP001262376, * the whole document * | 1 | |

-/--

TECHNICAL FIELDS
SEARCHED (IPC)

The present search report has been drawn up for all claims

| Place of search | Date of completion of the search | Examiner |
|---|---|---|
| The Hague | 21 December 2016 | Eberle, Katja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 16 17 7569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 249 004 B1 (MILLER STEVEN DONALD [US]) 19 June 2001 (2001-06-19) * column 3, line 42 - column 4, line 34; figures * ----- | 1 | |
| A | GB 1 140 028 A (ISOTOPES INC) 15 January 1969 (1969-01-15) * page 2, line 84 - line 113 * * page 3, line 99 - line 105 * ----- | 9,11, 13-15 | |

TECHNICAL FIELDS
SEARCHED (IPC)

The present search report has been drawn up for all claims

| Place of search | Date of completion of the search | Examiner |
|---|---|---|
| The Hague | 21 December 2016 | Eberle, Katja |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 16 17 7569

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2016

| Patent document cited in search report | | Publication date | Patent family member(s) | | Publication date |
|---|---|---|---|---|---|
| US 6249004 | B1 | 19-06-2001 | NONE | | |
| GB 1140028 | A | 15-01-1969 | BE | 677480 A | 01-08-1966 |
| | | | CH | 433516 A | 15-04-1967 |
| | | | DE | 1539743 B1 | 22-10-1970 |
| | | | GB | 1140028 A | 15-01-1969 |
| | | | NL | 6603030 A | 12-09-1966 |
| | | | US | 3471699 A | 07-10-1969 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **E.G. YUKIHARAA et al.** *Radiation Measurements,* 2008, vol. 43, 309-314
- **GRANVILLE et al.** *Physics in Medicine and Biology,* 2016, vol. 61, 1765-1779
- **M.S. AKSELROD et al.** *Radiation Measurements,* 2007, vol. 41, S78-S99